# EUROPEAN PATENT APPLICATION

(11) **EP 4 173 614 A1**
(43) Date of publication of application: **03.05.2023**
(21) Application number: 21833046.2
(22) Date of filing: 29.06.2021
(51) Int. Cl.: A61K 9/00, A61K 9/127, A61K 47/14, A61K 47/24, A61K 47/22, A61K 47/28, A61K 38/09, A61P 5/02

(54) **INJECTABLE COMPOSITION COMPRISING GNRH DERIVATIVES**

(30) Priority: 30.06.2020 KR 20200080621
(71) Applicant: Chong Kun Dang Pharmaceutical Corp., Seoul 03742 (KR)
(72) Inventor: KO, Jin Young, Yongin-si Gyeonggi-do 16995 (KR); JUNG, Sung Bum, Yongin-si Gyeonggi-do 16995 (KR); NOH, Sang Myoung, Yongin-si Gyeonggi-do 16995 (KR); LIM, Jong Lae, Seoul 03742 (KR)
(74) Representative: D Young & Co LLP
(86) International application number: PCT/KR2021/008187
(87) International publication number: WO 2022/005169

(57) **Abstract**

An injectable composition of the present invention has remarkably increased safety by forming, into a composition, a sorbitan unsaturated fatty acid ester comprising g a polar head group with two or more -OH (hydroxyl) groups, is in a liquid phase in a phase where an aqueous fluid is absent so as to be easily applied to pharmaceutical preparations in dosage forms, and forms a liquid crystal in an aqueous fluid phase so that sustained-release effects of GnRH analogue, which is used as a pharmaceutically active substance in vivo, are exhibited. In addition, in an injectable composition of the present invention, a sustained-release lipid initial preparation comprising GnRH analogue includes water, and thus a liquid injectable agent forms a liquid crystal gel immediately after administration, so that the effects of reducing initial release rate and remarkably increasing sustained-release properties of the drug are exhibited.

## Description

### Technical Field

The present invention relates to a sustained-release depot formulation including GnRH analogue as a pharmaceutically active substance and an injectable composition including the same.

### Background

A sustained-release formulation is a preparation which may continuously release a pharmacologically active substance with a single administration so as to prevent side effects likely to occur during repeated administrations and maintain an effective concentration range of the pharmacologically active substance for a certain time or for a certain period of time or more.

Considering the therapeutic mechanism and physicochemical properties of the pharmacologically active substance, a representative pharmacologically active substance suitable to be designed in the form of a sustained-release preparation includes GnRH analogues.

Gonadotropin-releasing hormone (GnRH) or luteinizing hormone releasing hormone (LHRH) is a neuroendocrine peptide synthesized at a neurovascular end of the hypothalamus. The GnRH is secreted from the hypothalamus, selectively binds to a specific receptor on a membrane of an anterior pituitary gonadotroph cell, and then stimulates the biosynthesis and secretion of luteinizing hormone (LH) and follicle-stimulating hormone (FSH). The produced follicle-stimulating hormone (FSH) and the luteinizing hormone (LH) play a role in regulating the production of sex steroids in the male and female gonads. Due to the biological functions of the GnRH, the GnRH analogues may be used in the treatment of sex hormone-dependent diseases such as prostate cancer, breast cancer, ovarian cancer, endometriosis, uterine fibroid, polycystic ovarian disease, hypertrichosis, precocious puberty, gonadotroph pituitary adenomas, sleep apnea syndrome, irritable bowel syndrome, premenstrual syndrome, benign prostatic hyperplasia, or infertility, or the like.

Among the widely marketed products, the sustained-release products of GnRH analogues include Lupron^{®} Depot, which contains leuprolide acetate as a pharmacologically active substance. Lupron^{®} Depot is widely used as an intramuscular and subcutaneous injectable agent using biodegradable poly(lactic-co-glycolic acid) (PLGA) microspheres as a sustained-release substrate. In general, the PLGA microspheres stay in the body for a certain period of time and are decomposed into lactic acid and glycolic acid to continuously release an encapsulated pharmacologically active substance, thereby exhibiting a sustained-release effect (US Patent No. 5,480,656). However, a process of preparing the PLGA microspheres has disadvantages in that it is complicated and difficult, and the encapsulation efficiency of the pharmacologically active substance is significantly lowered. In addition, the PLGA microspheres are difficult to be filtered and they also melt at a temperature of 40°C or higher, and thus it is impossible to apply the method generally used for aseptic treatment. Accordingly, there is a difficulty in that the process needs to be performed under highly aseptic conditions. Moreover, it is necessary to further perform a complicated process of preparing and mixing two or more different microspheres in order to create an ideal sustained-release pattern (International Publication No. WO 2005/074896), and thus economic costs are increased due to the inefficient process. Furthermore, acetic acid impurities and acid decomposition substances from the PLGA microspheres cause an inflammatory reaction, reduction of cell proliferation, etc. (K. Athanasiou, G. G. Niederauer, and C. M. Agrawal, Biomaterials, 17, 93 (1996)). Due to the nature of a product, which requires a large amount of intramuscular and subcutaneous administration by suspending microspheres with a size of 10 to 100 µm in an aqueous solution, there is a problem in which pain or tissue damage occurs at an injection site. A sustained-release injectable agent of the GnRH analogue (leuprolide acetate) introduced to compensate for the shortcoming of the PLGA microsphere sustained-release formulation includes ELIGARD Inj^{®}. ELIGARD Inj is widely marketed as a subcutaneous injectable agent which is used by dissolving poly(DL-lactide-co-glycolide) having a protected carboxyl terminal group and GnRH analogue (leuprolide acetate) in N-methyl-2-pyrrolidone (NMP). ELIGARD Inj^{®} has addressed some of the disadvantages of the solid PLGA microsphere formulation by dissolving a biodegradable polymer in a polar aprotic solution to prepare a flowable composition and then subcutaneously administering the same (US Patent No. 6,773,714). However, the product does not provide a complete pre-filled injection device, and thus has disadvantages in that it is very less convenient and has low drug stability in a mixed solution state. A kit provided by the product consists of two connectable syringes and devices for mixing, preparation and injection. In detail, a complicated process of about 10 steps or more is required to prepare a final mixed solution, and there is a difficulty in completing the preparation and administration within 30 minutes. In addition, the product requires a separate refrigeration storage device due to storage conditions, and if the final mixed solution is not refrigerated, the product cannot be used for five days or longer. In addition, the formulation has failed to address a high initial drug release phenomenon, which is a general disadvantage of PLGA formulations, but rather has showed a higher initial drug concentration than Lupron^{®} Depot, a PLGA microsphere formulation (US Patent No. 6,773,714). The initial drug concentration significantly above a range in which a drug can work is not functionally or toxicologically desirable. In particular, considering that GnRH analogues have a mechanism in which sex hormone secretion is temporarily increased at the beginning of administration and then downregulated after a certain point in time, an excessive initial drug release is a factor that must be avoided.

As an alternative to overcome the problems of these PLGA formulations, International Publication No. WO 2005/117830 discloses an initial preparation which includes at least one neutral diacyl lipid and/or tocopherol, at least one phospholipid, and at least one organic solvent which is biocompatible, contains oxygen, and has a low viscosity. International Publication No. WO 2006/075125 discloses an initial preparation which includes at least one diacyl glyceride, at least one phosphatidylcholine, at least one oxygen-containing organic solvent, and at least one GnRH analogues. These preparations do not produce lactic acid or glycolic acid degradation products based on a polymer system, and thus do not involve pain or inflammation at an injection site, but continuously release pharmacologically active substances in vivo for about four weeks. However, diacyl lipid, which is a key component of the preparation composition, is a material which is not generally used as an excipient for pharmaceuticals due to the lack of sufficient safety and needs to use an organic solvent causing a decline in the activity of some pharmacologically active substances (H. Ljusberg-Wahre, F. S. Nielse, 298, 328-332 (2005); H. Sah, Y. bahl, Journal of Controlled Release 106, 51-61(2005)).

Accordingly, the present inventors have invented a pharmaceutical composition, which includes: a) sorbitan unsaturated fatty acid ester; b) phospholipid; c) a liquid crystal hardener; and d) GnRH analogues as a pharmacologically active substance, is a lipid liquid in an absence of an aqueous fluid, and forms liquid crystals in an aqueous fluid (Korean Patent Publication No. 10-2012-0157583). It has been confirmed that a sustained-release lipid initial preparation according to the present invention has safety and biodegradability equal or superior to those of an existing initial preparation in an animal test (in vivo), and a pharmaceutical composition including a pharmacologically active substance releases a drug in a sustained manner. However, in the sustained-release lipid initial preparation of the present invention, a drug release was terminated within two weeks, contrary to expectations in clinical (human). This is because the sustained-release lipid initial preparation spreads to the subcutaneous fat or tissues in the human body with a low moisture content and a high-density subcutaneous fat, and thus a liquid crystal gel is spread or created in a fragmented form, resulting in early termination of the sustained release of the drug.

Accordingly, the present inventors have repeatedly conducted a research by adding water in advance to the sustained-release lipid initial preparation containing the GnRH analogues, and thus have completed an invention on a sustained-release depot preparation with a remarkably improved initial release rate and a sustained release property of the drug as a liquid injectable agent forms a liquid crystal gel immediately after administration.

Hereinafter, prior art which may relate to the present invention has been examined.

U.S. Patent No. 7,731,947 discloses a composition in which solid particles composed of interferon, sucrose, methionine, and citrate buffer are dispersed in an organic solvent such as benzyl benzoate, etc., and explains that GnRH analogues may be used as an applicable drug. In addition, in some examples, it is described that phosphatidylcholine may be dissolved in an organic solvent together with vitamin E (tocopherol) and used as a dispersion of solid particles. However, the composition of the above patent is different from that of the present invention in that liquid crystals are not formed, but the composition is used for dispersing solid particles.

U.S. Patent No. 7,871,642 discloses a method for preparing a dispersion in which a mixture of phospholipid, a surfactant having polyoxyethylene, triglyceride, and ethanol compositions is dispersed in water, so as to deliver a pharmacologically active substance including a hormone agent, and also explains that polyoxyethylene sorbitan fatty acid ester (polysorbate) and polyoxyethylene vitamin E derivatives may be used as one of the surfactants having polyoxyethylene. However, polyoxyethylene sorbitan fatty acid ester and polyoxyethylene vitamin E derivatives are substances, in which polyoxyethylene, a hydrophilic polymer, is bonded with sorbitan fatty acid ester and vitamin E, respectively, and are also substances which have a structure completely different from that of the original sorbitan fatty acid ester and vitamin E and are used as a hydrophilic surfactant using the properties of polyoxyethylene, and thus are different from the constituent components of the present invention.

U.S. Patent No. 5,888,533 discloses a composition which provides a sustained release property of pharmacologically active substances including leuprolide, which includes a substance that is non-polymeric, water-insoluble, and biodegradable as a fluid composition for forming an implant, and a solvent that at least partially dissolves the substance and is miscible or dispersible with water or biological fluids, and in which the solvent diffuses and escapes into the biological fluid when applied to the human body, so that a non-polymeric, water-insoluble, and biodegradable substance aggregates or precipitates so as to form an implant. Here, it is described that sterol, cholesteryl ester, fatty acid, fatty acid glyceride, sucrose fatty acid ester, sorbitan fatty acid ester, fatty alcohols, ester bond products of fatty alcohol and fatty acid, dehydrated product of fatty acid, phospholipid, lanolin, lanolin alcohol, etc. may be used as the non-polymeric, water-insoluble, and biodegradable substance, and ethanol. etc., are described as a solvent. However, the above patent is different from the present invention not only in that it involves a composition for preparing an implant through simple agglomeration or precipitation without liquid crystals formed, but also in that a large amount of an organic solvent is necessarily used.

International Publication No. WO 2012/160212 discloses an initial preparation which includes 20-80% of at least one diacyl lipid and/or tocopherol, 20-80% of at least one phospholipid, 5-35% of at least one biocompatible organoalcoholic solvent, 20% or less of aqueous solution, and at least one dual amylin receptor/GLP-1 receptor agonist drug, and US Application No. 20070265190 discloses an initial preparation which includes at least one diacyl lipid and/or tocopherol, at least one phospholipid, at least one oxygen-containing low-viscosity organic solvent, and at least one opioid drug. These preparations do not produce lactic acid or glycolic acid degradation products based on a polymer system, and thus do not involve any pain or inflammation at an injection site, but continuously release pharmacologically active substances in vivo as a drug. However, diacyl lipid, which is a key component of the preparation composition, is a substance which is not generally used as an excipient for pharmaceuticals due to the lack of sufficient safety, and has failed to prove a drug release behavior according to moisture in the same conditions as in the human body with developed subcutaneous fat. In addition, the composition is different from that of the present invention in that it is a composition having a sustained-release property of a drug completely different from the drug of the present invention.

International Publication No. WO 2005/074896 discloses a composition which releases a GnRH agonist or salts thereof for a long period of time as PLGA microcapsules containing an aqueous solution. In addition, the patent describes maintaining a low side effect and a stable sustained-release property for drug dumping with zero order-release without an initial drug dumping phenomenon. However, the composition of the patent is different from that of the present invention in that the composition is a sustained-release formulation which is completely different from that of the present invention as a PLGA microparticle formulation, and an aqueous channel inside the PLGA microparticles is used for containing a water-soluble drug.

### [Related Art Reference]

### [Patent Documents]

(Patent Document 1) International Publication No. WO 2005/074896
(Patent Document 2) International Publication No. WO 2005/117830
(Patent Document 3) International Publication No. WO 2006/075125
(Patent Document 4) International Publication No. WO 2012/160212
(Patent Document 5) U.S. Patent No. 5,480,656
(Patent Document 6) U.S. Patent No. 6,773,714
(Patent Document 7) U.S. Patent No. 7,731,947
(Patent Document 8) U.S. Patent No. 7,871,642
(Patent Document 9) U.S. Patent No. 5,888,533
(Patent Document 10) U.S. Application No. 20070265190

### Detailed Description of the Invention

### Technical Problem

An object of the present invention is to provide an injectable composition which has remarkably increased safety by forming, into a composition, a sorbitan unsaturated fatty acid ester having a polar head with two or more -OH (hydroxyl) groups, is in a liquid phase in a phase where an aqueous fluid is absent so as to be easily applied to pharmaceutical preparations in dosage forms, and forms a liquid crystal in an aqueous fluid phase so that a sustained release property of GnRH analogues, which are used as a pharmaceutically active substance in vivo, can be increased.

An object of the present invention is to provide an injectable composition which has remarkably increased safety by forming, into a composition, a sorbitan unsaturated fatty acid ester having a polar head with two or more -OH (hydroxyl) groups, is in a liquid phase in a phase where an aqueous fluid is absent so as to be easily applied to pharmaceutical preparations in dosage forms, and forms a liquid crystal in an aqueous fluid phase so that a sustained release property of GnRH analogues, which are used as a pharmaceutically active substances in vivo, can be increased.

Another object of the present invention is to provide an injectable composition which can be injected in a liquid phase and thus can ameliorate injection pain, inflammation, and high initial release concentration, which have not been overcome by conventional sustained-release preparation.

### Technical Solution

The present invention provides an injectable composition which includes: a) a sorbitan unsaturated fatty acid ester comprising a polar head with two or more -OH (hydroxyl) groups; b) phospholipid; c) a liquid crystal hardener which does not have an ionized group, and of which a hydrophobic portion has a triacyl group comprising 15 to 40 carbon atoms or a carbon ring structure; d) water; and e) a gonadotropin-releasing hormone (GnRH) analogue as a pharmaceutically active substance, and in which the composition is liquid before injection and forms a liquid crystal after injection.

Hereinafter, each component will be described in detail.

### a) Sorbitan unsaturated fatty acid ester

Sorbitan unsaturated fatty acid ester, which is a liquid crystal forming agent of the present invention, may refer to a compound of [Formula 1] comprising a polar head with two or more -OH (hydroxyl) groups, among which sorbitan monoester is R¹=R²=OH, R³=R, and sorbitan diester is R¹=OH, R²=R³=R or R³=OH, R¹=R²=R. Here, R refers to an alkyl ester group comprising 4 to 30 carbon atoms and at least one double bond.

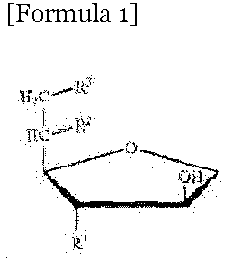

This is different from a known fact that a liquid crystallization phenomenon is unusually caused by oleyl glycerate (OG), phytanyl glycerate (PG), glycerine monooleate (GMO), and glycerin dioleate (GDO), which are represented by [Formula 2], in the related art. In other words, the conventionally known substances for forming liquid crystals have a structure in common that they all have a polar head composed of glycerine or glyceric acid, which is bonded with a nonpolar tail derived from the form of fatty alcohols or fatty acids.

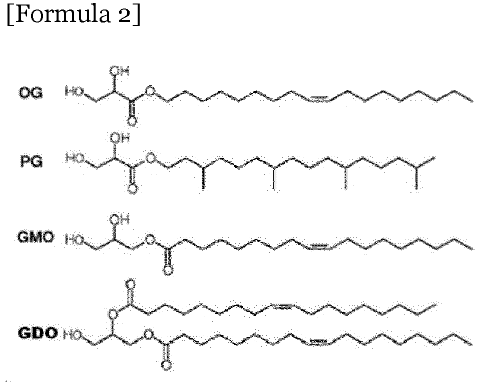

However, the conventional substances for forming liquid crystals are difficult to be applied to drug development due to each of the following disadvantages. Oleyl glycerate (OG) and phytanyl glycerate (PG) may form liquid crystals well, but are not used as excipients for pharmaceuticals due to relatively high toxicity. In contrast, glycerin monooleate (GMO) may be used as a pharmaceutical excipient, but has a disadvantage in that it could not form any sustained-release liquid crystal required for pharmaceuticals due to low ability to form liquid crystals.

In addition, glycerin dioleate (GDO) of International Publication No. WO 2005/117830 described above uses glycerin as a polar head portion in the form of a glyceride having a diacyl group, but has a problem in that it is not generally used as an excipient for pharmaceuticals due to the lack of sufficient safety.

The present inventors have found that, unlike conventional glycerin or glyceric acid derivatives, liquid crystals formed by sorbitan unsaturated fatty acid esters are not only advantageous for sustained release of an active substance, but also have superior safety compared to conventional liquid crystal forming substances, thus overcoming the shortcomings of the conventional technology and being available for drug development. In order to be used as a pharmaceutical composition, safety must be secured and biodegradability is an essential element. Moreover, in the case of a substance which is injected to in vivo and exhibits a sustained release property, biodegradability is a very important factor. In the case of the PLGA, which is used as a conventional sustained-release injectable agent, if the PLGA shows a sustained-release effect for one week, it is desirable that the injected PLGA is decomposed in vivo and disappears ideally in one week later. However, there is a problem that the PLGA actually persists without decomposition for a period ranging from one month to several months even after completion of a sustained-release function. Thus, it is clear that the sorbitan unsaturated fatty acid ester of the present invention has an excellent sustained-release property, safety, and biodegradability, and thus may be a new liquid crystal forming substance having a very high industrial value.

Specifically, sorbitan unsaturated fatty acid ester of the present invention may be at least one selected from sorbitan monoester, sorbitan sesquiester, sorbitan diester, and mixtures thereof derived from fatty acids which may be obtained from vegetable oils (e.g., palm oil, castor oil, olive oil, peanut oil, rapeseed oil, corn oil, sesame oil, cottonseed oil, soybean oil, sunflower oil, safflower oil, linseed oil, etc.), animal fats and oils (e.g., milk fat, pork fat and beef tallow) as well as whale oil and fish oil.

The sorbitan monoester may be one in which one fatty acid group is ester-bonded to sorbitan, and may be at least one selected from sorbitan monooleate, sorbitan monolinoleate, sorbitan monopalmitoleate, sorbitan monomyristoleate, and mixtures thereof.

The sorbitan sesquiester may be one in which 1.5 fatty acid groups on average are ester-bonded to sorbitan, and may be at least one selected from sorbitan sesquioleate, sorbitan sesquilinoleate, sorbitan sesquipalmitoleate, sorbitan sesquimyristoleate, and mixtures thereof.

The sorbitan diester may be one in which two fatty acid groups are ester-bonded to sorbitan, and may be selected from sorbitan dioleate, sorbitan dilinoleate, sorbitan dipalmitoleate, sorbitan dimyristoleate, and mixtures thereof.

The sorbitan unsaturated fatty acid ester according to the present invention may be preferably selected from sorbitan monooleate, sorbitan monolinoleate, sorbitan monopalmitoleate, sorbitan monomyristoleate, sorbitan sesquioleate, and mixtures thereof.

### b) Phospholipid

The phospholipid of the present invention is a substance which has been essentially used for the preparation of a lamellar structure such as a liposome in the related art, but could not independently form liquid crystals as a non-lamellar phase structure. However, in the present invention, the phospholipid plays a role in stabilizing liquid crystals by participating in the non-lamellar structure triggered by the sorbitan unsaturated fatty acid ester, which is the liquid crystal forming agent of the present invention.

The phospholipid of the present invention is a form derived from plants or animals, specifically have an alkyl ester group comprising 4 to 30 saturated or unsaturated carbon atoms, and which is at least one selected from phosphatidylcholine, phosphatidylethanolamine, phosphatidylserine, phosphatidylglycerine, phosphatidylinositol and phosphatidic acid, sphingomyelin, and mixtures thereof according to a structure of a polarity head.

And, the phospholipid is a form derived from plants or animals, such as soybean, egg, or the like, and the alkyl ester group bonded to the phospholipid may include saturated fatty acid esters such as mono- and dipalmitoyl, mono- and dimyristoyl, mono- and dilauryl, mono- and distearyl, etc., or unsaturated fatty acid esters such as mono- and dilinoleyl, mono- and dioleyl, mono- and dipalmitoleyl, mono- and dimyristoleyl, and the like, or may include a form in which saturated fatty acid esters and unsaturated fatty acid esters are present together.

### c) Liquid crystal hardener

The liquid crystal hardener of the present invention could not independently form a non-lamellar structure like a liquid crystal forming agent, and also could not form a layered structure such as liposome like phospholipid. However, the liquid crystal hardener of the present invention may participate in the non-lamellar structure triggered by the liquid crystal forming agent so as to increase a curvature (distortion) of the non-lamellar structure, resulting in further increasing a degree of regular mixing of oil and water. In order to have such function as the liquid crystal hardener, it is advantageous that polarity is very limited in a molecular structure and a non-polar region is bulky at the same time.

However, unlike the above, in the case of the liquid crystal hardener of the present invention, it is very unusual that a substance which is injectable to the human body and biocompatible may be selected only through direct and repeated experiments. As a result, each of the liquid crystal hardeners suitable for the composition of the present invention has different molecular structures from each other, and cannot be explained by a single structure. However, when finding a liquid crystal hardener suitable for the composition of the present invention and then observing a structure thereof, it could be confirmed that the liquid crystal hardener does not have an ionized group such as a carboxyl group or an amine group, and a hydrophobic portion has a bulky triacyl group with 15 to 40 carbon atoms in total or has a carbon ring structure. Preferably, the liquid crystal hardener does not have an ionized group such as a carboxyl group or an amine group, and has at most one hydroxyl group and an ester structure as a weakly polar portion, and a relatively hydrophobic portion has a bulky triacyl group having 20 to 40 carbon atoms in total or has a carbon ring structure. Thus, specifically, the liquid crystal hardener of the present invention may be at least one selected from triglyceride, retinyl palmitate, tocopherol acetate, cholesterol, benzyl benzoate, ubiquinone, and mixtures thereof, but is not limited thereto. Preferably, the liquid crystal hardener of the present invention may be tocopherol acetate, cholesterol, or mixtures thereof.

### d) Water

The water of the present invention serves to rapidly form a liquid crystal gel immediately after administration of a liquid injectable agent. A lipid initial preparation which does not contain water may draw moisture in vivo after injection and form a liquid crystal gel very slowly. As the lipid initial preparation forms the liquid crystal gel at a slow rate, the lipid initial preparation may widely spread in an injection site and form the liquid crystal gel at that state, thereby forming gel with a large surface area or fragmented gel. A liquid crystal gel which has a large surface area or is fragmented may cause a dumping phenomenon of a drug at an initial stage of administration, which results in an insufficient amount of drug release in a late stage of a sustained release period, thus making it difficult to maintain a stable sustained release property for a long period of time. In contrast, in the present invention, a lipid initial preparation contains water, and thus form gel immediately upon administration into tissues so as to lower an initial release rate of a drug, thereby providing a stable sustained release of the drug.

In the present invention, water maybe added as water for injection, distilled water, buffer, or mixtures of two or more selected therefrom.

### e) GnRH analogues

GnRH analogues are structurally similar to GnRH, but differently act in the body. In general, the GnRH performs a biological function of inducing the production of sex steroids in the body through pulsatile secretion, but the GnRH analogues are used to strongly inhibit the production of sex steroids in the body for a certain period of time.

These GnRH analogues are classified into an agonist or an antagonist according to a mechanism of action. When a therapeutic dose of the GnRH agonist is administered into the body, the GnRH agonist initially binds to a GnRH receptor of pituitary gonadotropin and promote the biosynthesis and secretion of follicle-stimulating hormone (FSH) and luteinizing hormone (LH). However, continuous administration of the GnRH agonist depletes gonadotropin, while down-regulating the GnRH receptor, thereby inhibiting the biosynthesis and secretion of the follicle-stimulating hormone (FSH) and the luteinizing hormone (LH). Due to the biological functions of the GnRH, the GnRH analogues are used in the prevention or treatment of sex hormone-dependent diseases such as prostate cancer, breast cancer, ovarian cancer, endometriosis, uterine fibroid, polycystic ovarian disease, hypertrichosis, precocious puberty, gonadotroph pituitary adenomas, sleep apnea syndrome, irritable bowel syndrome, premenstrual syndrome, benign prostatic hyperplasia, or infertility, or the like, and may be used as a contraceptive.

The GnRH agonist, which may be used as a pharmacologically active substance of the present invention, may be selected from leuprolide, goserelin, triptorelin, nafarelin, buserelin, histrelin, deslorelin, meterelin, gonadrelin, or pharmacologically acceptable salts thereof. Preferably, the GnRH agonist may be at least one pharmacologically active substance selected from leuprolide, goserelin, and pharmacologically acceptable salts thereof.

In contrast, a GnRH antagonist competitively responds to the GnRH receptor of pituitary gonadotropin so as to block GnRH binding in the body, thereby inhibiting the biosynthesis and secretion of the follicle-stimulating hormone (FSH) and the luteinizing hormone (LH). The GnRH antagonist, which may be used as the pharmacologically active substance of the present invention, may be selected from degarelix, abarelix, ganirelix, cetrorelix, and pharmacologically acceptable salts thereof. Preferably, the GnRH antagonist may be at least one pharmacologically active substance selected from degarelix and pharmacologically acceptable salts thereof.

Meanwhile, the injectable composition of the present invention may further include a solvent in addition to a) to e). Examples of the solvent may include ethanol, dimethyl sulfoxide (DMSO), benzyl alcohol, benzyl benzoate, methylpyrrolidone, propylene glycol, or the like, which may be used alone or in combination of two or more thereof.

A weight ratio of component a) and component b) suitable for the desired liquid crystal of the injectable composition of the present invention may be 10:1 to 1:10, and specifically 5:1 to 1:5. A weight ratio of components a) + b) and component c) may be 100:1 to 1:1, and may be, for example, 50:1 to 2:1. Within the above range, a sustained-release effect by desired the liquid crystal of the present invention may be good, and it may be possible to control a sustained-release pattern by controlling a ratio thereof.

In addition, a weight ratio of components a) + b) + c) and component d) suitable for the desired liquid crystal of the injectable composition of the present invention may be 99:1 to 1:1, and maybe, for example, 99:1 to 90:10 or 75:25 to 62.5:37.5.

Furthermore, a weight ratio of components a) + b) + c) + d) and component e) suitable for the desired liquid crystal of the injectable composition of the present invention may be 10000:1 to 1:1, and may be, for example, 1000:1 to 1:1.

In the present invention, a content of one component expressed as "wt%" may mean a percent, for which a weight of the component accounts when the total weight of the injectable composition is 100%. The injectable composition of the present invention may include an extra solvent together with components a) to e).

The injectable composition of the present invention may include: 9 to 90 wt% of the component a); 9 to 90 wt% of the component b); 0.1 to 50 wt% of the component c); 0.5 to 50 wt% of the component d); and 0.01 to 50 wt% of the component e).

In one example, the injectable composition of the present invention may include: 9 to 50 wt% of the component a); 18 to 60 wt% of the component b); 1 to 36 wt% of the component c); 0.5 to 50 wt% of the component d); and 0.1 to 45 wt% of the component e).

In one example, the injectable composition of the present invention may include: 9 to 50 wt% of the component a); 18 to 60 wt% of the component b); 1 to 36 wt% of the component c); 0.5 to 10.5 wt% of the component d); and 0.1 to 45 wt% of the component e).

In one example, the injectable composition of the present invention may include: 9 to 50 wt% of the component a); 18 to 60 wt% of the component b); 1 to 36 wt% of the component c); 2.5 to 10.5 wt% of the component d); and 0.1 to 45 wt% of the component e).

In one example, the injectable composition of the present invention may include: 9 to 50 wt% of the component a); 18 to 60 wt% of the component b); 1 to 36 wt% of the component c); 25 to 37.5 wt% of the component d); and 0.1 to 45 wt% of the component e).

In one embodiment of the present invention, if the pharmacologically active substance is leuprolide, the injectable composition of the present invention may include: 9 to 50 wt% of the component a); 18 to 60 wt% of the component b); 1 to 36 wt% of the component c); 0.5 to 10.5 wt% or 25 to 37.5 wt% of the component d); and 0.1 to 45 wt% of leuprolide e) or pharmaceutically acceptable salts thereof.

In one embodiment of the present invention, if the pharmacologically active substance is goserelin, the injectable composition of the present invention may include: 9 to 50 wt% of the component a); 18 to 60 wt% of the component b); 1 to 36 wt% of the component c); 0.5 to 10.5 wt% or 25 to 37.5 wt% of the component d); and 0.1 to 45 wt% of goserelin e) or acceptable salts thereof for injection.

In one embodiment of the present invention, if the pharmacologically active substance is degarelix, the injectable composition of the present invention may include: 9 to 50 wt% of the component a); 18 to 60 wt% of the component b); 1 to 36 wt% of the component c); 0.5 to 10.5 wt% or 25 to 37.5 wt% of the component d); and 0.1 to 45 wt% of degarelix e) or pharmaceutically acceptable salts thereof.

When the injectable composition of the present invention contains components a) to e) in the above content, each of the pharmacologically active substances may have an excellent sustained-release effect.

In the present invention, the liquid crystal has a non-lamellar structure in which oil and water are regularly mixed and arranged under very limited conditions so that an internal phase and an external phase may not be distinguished from each other, and this non-lamellar structure has the properties of a mesophase between a liquid phase and a solid phase due to a unique oil-water regular arrangement. This has a different structure from one in which micelle, emulsion, microemulsion, liposome, lipid bilayer, etc., which have been widely used in the design of formulations for injection, all share the properties of a lamellar structure in common, and this lamellar structure is formed by dividing an inner phase and an outer phase in the form of oil-in-water (o/w) or water-in-oil (w/o).

In the present invention, a liquid crystallization phenomenon representing "liquid crystal" refers to a phenomenon in which a liquid crystal, a non-lamellar phase structure, is formed by exposure to an excess of aqueous fluid from the initial preparation as described above. The "excess" of the aqueous fluid in which the liquid crystallization phenomenon occurs may mean at least one or more times the total weight or volume of the injectable composition of the present invention.

The injectable composition of the present invention may be prepared at room temperature by adding a) at least one selected from sorbitan unsaturated fatty acid esters, b) at least one selected from phospholipids, c) at least one selected from liquid crystal hardeners, d) at least one selected from water for injection, distilled water, and buffer, and e) at least one selected from GnRH analogues, and may be prepared by applying heat or using a homogenizer, if necessary. In this case, the homogenizer used herein may be selected from a high-pressure homogenizer, an ultrasonic homogenizer, a crushing homogenizer, and the like.

The injectable composition of the present invention may be administered in any form of subcutaneous injection or intramuscular injection as an administration route of an injectable agent, and a dosage form may be selected depending on the properties of each pharmacologically active substance.

The injectable composition of the present invention may be prepared by adding a pharmacologically active substance to the initial preparation of the present invention at room temperature, and may be prepared by applying heat or using a homogenizer if necessary, but the present invention is not limited thereto.

In addition, the present invention may provide a method for preventing or treating sex hormone-dependent diseases, or for contraception, which comprises: administering a therapeutically effective amount of the injectable composition of the present invention into a subject.

In the present invention, the term "subject" may refer to mammals including humans, and the term "administration" may refer to providing a predetermined material to a subject through any appropriate method.

The "therapeutically effective amount" of the injectable composition of the present invention may refer to an amount of an active substance or a pharmaceutical composition which induces animals or humans to show the biological or medical responses considered by investigators, veterinarians, doctors or clinicians. The therapeutically effective amount may include an amount thereof for inducing a relief of symptoms of diseases or disorders to be treated, may be the same as the known dosage of the pharmacologically active substance used herein, but may vary depending on a patient's disease type, degree of symptom, age, gender, etc., and may be administered in any form of subcutaneous injection or intramuscular injection depending on the properties of the pharmacologically active substance and the drug. The present invention may provide a use of the injectable composition according to the present invention for preventing or treating sex hormone-dependent diseases, or for contraception.

The present invention may provide a use of the injectable composition according to the present invention, in the manufacture of a medication for preventing or treating sex hormone-dependent diseases, or for contraception.

Matters mentioned in the injectable composition of the present invention, the use and therapeutic method thereof, and the sustained-release method of the pharmacologically active substance may be equally applied, if not contradictory to each other.

### Advantageous Effects

An injectable composition of the present invention can have remarkably increased safety by comprising a sorbitan unsaturated fatty acid ester having a polar head with two or more -OH (hydroxyl) groups, can be in a liquid phase in a phase where an aqueous fluid is absent so as to be easily applied to pharmaceutical preparations in dosage forms, and forms a liquid crystal in an aqueous fluid phase so that sustained-release effects of GnRH analogues, which are used as a pharmaceutically active ingredient in vivo, are exhibited. In addition, in the injectable composition of the present invention, a sustained-release lipid initial preparation including GnRH analogues includes water in advance, and thus a liquid injectable agent can form a liquid crystal gel immediately after administration, so that the effects of reducing an initial release rate and remarkably increasing a sustained-release property of the drug can be exhibited.

### Brief Description of the Drawings

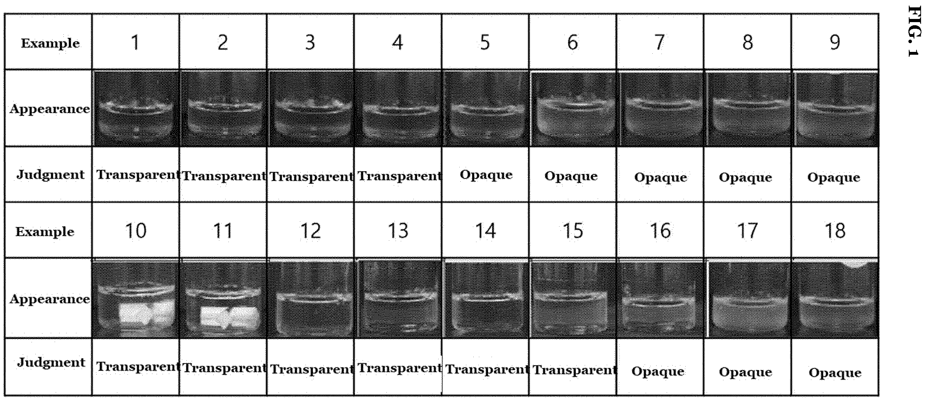

FIG. 1 is a view of pictures showing test results of identifying appearances of compositions according to Examples 1 to 18.
FIG. 2 is a view of pictures confirming a sol-gel conversion over time in the subcutaneous fat (ex-vivo) of pigs injected with the compositions of Comparative Examples 1 and Example 4, respectively.
FIG. 3 is a view of pictures identifying appearances of a gel in the mini pigs subcutaneously (in vivo) injected with the compositions of Comparative Example 2 and Example 10, respectively.
FIG. 4 is a graph showing a PK change up to 7 days in the guinea pigs each injected with the compositions of Comparative Example 3 and Example 13.
FIG. 5 is a graph showing a PK change up to 28 days in the guinea pigs each injected with the compositions of Comparative Example 3 and Example 13.
FIG. 6 is a graph showing a PK change of an initial drug release rate in the guinea pigs injected with the composition of Example 19.
FIG. 7 is a graph showing a PK change up to 28 days in the guinea pigs injected with the composition of Example 19.
FIG. 8 is a graph showing a PK change up to 7 days in humans each injected with the compositions of Comparative Example 3 and Example 13.
FIG. 9 is a graph showing a PK change up to 28 days in humans each injected with the compositions of Comparative Example 3 and Example 13.

### Mode for Invention

Hereinafter, the present invention will be described in more detail through the following examples and experimental examples. However, the following examples and experimental examples are provided only for the purpose of illustrating the present invention, and thus the scope of the present invention is not limited thereto.

The additives used in the present invention were the excipients according to the standards of the pharmacopoeia and a reagent purchased from Aldrich, Lipoid, Croda, and Seppic (company names).

### [Examples 1 to 18] Preparation of injectable composition of the present invention

Sorbitan unsaturated fatty acid ester, phospholipid, liquid crystal hardener, water, and pharmacologically active substances were added at the same weight as in Table 1 below.

Examples 1 to 18 were prepared as follows. First, leuprolide acetate, sorbitan monooleate, phosphatidylcholine, tocopherol acetate, DMSO and ethanol were mixed with a homogenizer (PowerGenmodel125. Fisher) at room temperature for 0.5 hours under the conditions of 1,000 to 3,000 rpm and homogenized to prepare a lipid solution. Then, a corresponding amount of water was added to the prepared lipid solution, and homogenized for about 5 to 30 minutes with a homogenizer under the conditions of about 1,000 to 3,000 rpm, so as to prepare an injectable composition in solution.

**[Table 1]**

| **Unit (mg)** | **Example** | | | | | | | | |
|---|---|---|---|---|---|---|---|---|---|
| | **1** | **2** | **3** | **4** | **5** | **6** | **7** | **8** | **9** |
| **Leuprolide acetate** | 3.75 | 3.75 | 3.75 | 3.75 | 3.75 | 3.75 | 3.75 | 3.75 | 3.75 |
| **Sorbitan monooleate** | 22.9 | 22.2 | 21.5 | 20.8 | 20.1 | 19.4 | 18.7 | 18.1 | 17.4 |
| **Phosphatidylcholine** | 45.7 | 44.3 | 43.0 | 41.6 | 40.2 | 38.8 | 37.5 | 36.1 | 34.8 |
| **Tocopherol acetate** | 15.2 | 14.8 | 14.3 | 13.9 | 13.4 | 13.0 | 12.5 | 12.0 | 11.6 |
| **DMSO** | 5 | 5 | 5 | 5 | 5 | 5 | 5 | 5 | 5 |
| **Ethanol** | 5 | 5 | 5 | 5 | 5 | 5 | 5 | 5 | 5 |
| **water** | 2.5 | 5 | 7.5 | 10 | 12.5 | 15 | 17.5 | 20.0 | 22.5 |

| **Unit (mg)** | **Example** | | | | | | | | |
|---|---|---|---|---|---|---|---|---|---|
| | **10** | **11** | **12** | **13** | **14** | **15** | **16** | **17** | **18** |
| **Leuprolide acetate** | 3.75 | 3.75 | 3.75 | 3.75 | 3.75 | 3.75 | 3.75 | 3.75 | 3.75 |
| **Sorbitan monooleate** | 16.7 | 16.0 | 15.3 | 14.7 | 13.9 | 13.3 | 12.6 | 11.9 | 11.2 |
| **Phosphatidylcholine** | 33.4 | 32.0 | 30.7 | 29.3 | 28.0 | 26.6 | 25.2 | 23.6 | 22.5 |
| **Tocopherol acetate** | 11.1 | 10.7 | 10.2 | 9.8 | 9.3 | 8.9 | 8.4 | 7.9 | 7.5 |
| **DMSO** | 5 | 5 | 5 | 5 | 5 | 5 | 5 | 5 | 5 |
| **Ethanol** | 5 | 5 | 5 | 5 | 5 | 5 | 5 | 5 | 5 |
| **water** | 25.0 | 27.5 | 30.0 | 32.5 | 35.0 | 37.5 | 40.0 | 42.5 | 45 |

### [Example 19] Preparation of injectable composition of the present invention

In Example 19, 292 mg of leuprolide acetate, 430 mg of DMSO, 1,034 mg of sorbitan monooleate, 2,195 mg of phosphatidylcholine, 697 mg of tocopherol acetate, and 452 mg of anhydrous alcohol were added to a glass vial and stirred at room temperature to prepare a transparent lipid solution. The prepared lipid solution was filtered through a PVDF Capsule Filter 0.2 *µ*m, and then the weight was measured. Water was injected into the measured lipid solution to obtain a transparent solution, in which a water content might account for 10.5% (w/w) based on 100% by weight of the transparent solution obtained by injecting water, then additionally stirred at room temperature to confirm the transparent solution, and then finally filtered through a PVDF Capsule Filter 0.2*µ*m so as to prepare an injectable liquid injectable formulation.

### [Comparative Examples 1 to 3]

Sorbitan unsaturated fatty acid ester, phospholipid, liquid crystal hardener, and pharmacologically active substance were added at the same weight as in Table 2 below.

In Comparative Examples 1 to 3, leuprolide acetate, sorbitan monooleate, phosphatidylcholine, tocopherol acetate, DMSO and ethanol were mixed with a homogenizer (PowerGenmodel125. Fisher) at room temperature for 0.5 hours under the conditions of 1,000 to 3,000 rpm and homogenized.

**[Table 2]**

| **Unit (mg)** | **Comparative Example** | | |
|---|---|---|---|
| | **1** | **2** | **3** |
| **Leuprolide acetate** | 3.75 | 3.75 | 3.75 |
| **Sorbitan monooleate** | 22.2 | 20.8 | 15.3 |
| **Phosphatidylcholine** | 44.3 | 41.6 | 30.7 |
| **Tocopherol acetate** | 14.8 | 13.9 | 10.2 |
| **DMSO** | 5 | 5 | 5 |
| **Ethanol** | 5 | 5 | 5 |
| **water** | - | - | - |

### [Experimental Example 1] Appearance Identification Test

The appearances of the formulations prepared through Examples 1 to 18 of the present invention were identification. It can be confirmed that the Examples 1 to 4 have transparent appearances when a water content is in the range of 0.5 to 10 wt% based on 100 wt% of the total composition. In addition, it can be confirmed that the appearances of Examples 10 to 15 having a water content of 25 to 37.5 wt% are transparent. This is a section in which water having different physical properties from lipids is stably dissolved without being separated or suspended in the lipid solution.

### [Experimental Example 2] Sol-Gel Conversion Test in pig subcutaneous fat (ex-vivo)

A sol-gel conversion test was performed in porcine subcutaneous fatty tissues with the compositions according to Comparative Example 1 and Example 4 of the present invention as follows.

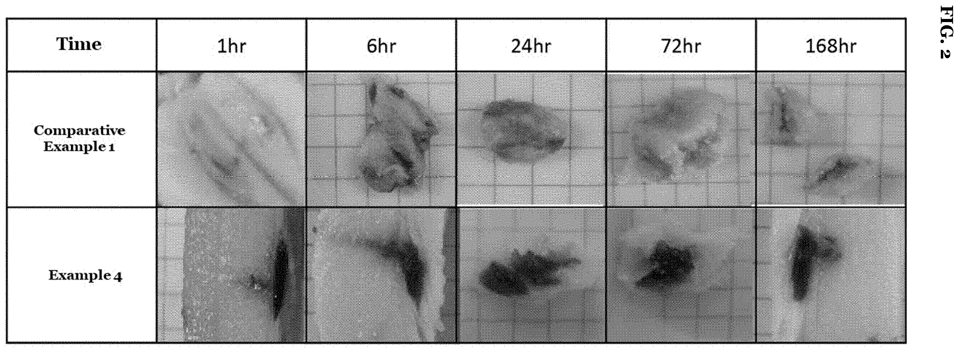

After slowly injecting 100 uL of the compositions according to Comparative Example 1 and Example 4 into porcine subcutaneous fat, the injection site was cut at 1, 6, 24, 72 and 168 hours after injection to observe a cross-section thereof. The results thereof are shown in [FIG. 2].

Referring to FIG. 2, in the case of Comparative Example 1 in which water was not added in the porcine subcutaneous fatty tissues, it could be confirmed that the liquid formulation sucks moisture from the tissues and is very slowly converted into a gel. In the case of Example 4 containing water, it could be confirmed that the liquid formulation already contains water, and thus is rapidly converted into a gel immediately after administration.

As in Comparative Example 1, a drug is rapidly released in a sol state in the body. In the examples according to the present invention, the drug passes through numerous lipid lattice structures and thus is released very slowly after being converted into a gel having a liquid crystal structure.

### [Experimental Example 3] Appearance Identification Test of gel in mini pig under the skin (in vivo)

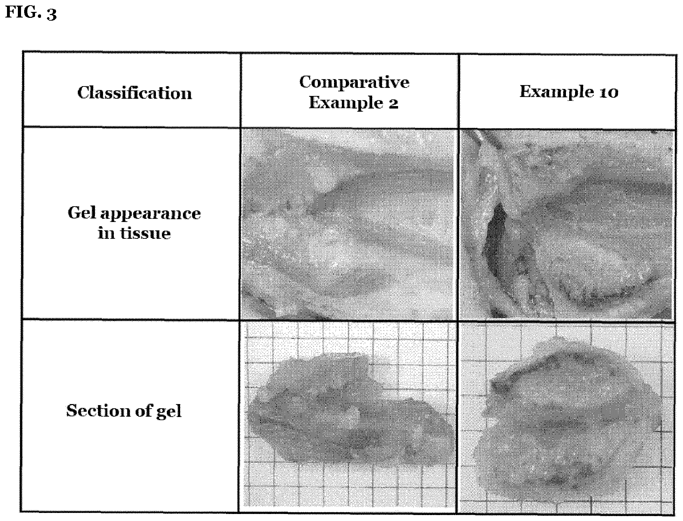

For Comparative Example 2 and Example 10 of the present invention, the gel appearances in the subcutaneous tissues of a mini pig were identification. The experiment was performed by administering 0.3 mL each of the compositions of Comparative Example 2 and Example 10 by using a disposable syringe under the back skin of a mini pig (male) weighing about 15 kg. In seven days after the administration, an administration site was excised, and the results of comparative observation of the gel appearances are shown in FIG. 3.

Referring to FIG. 3, the composition according to Comparative Example 2 is a lipid liquid formulation without addition of water, and when converted from sol to gel, the composition is converted to gel at a very slow rate while slowly drawing in body fluid in the tissue. Because the composition is converted to gel very slowly, the sol that has not become a gel permeates between the subcutaneous fats and forms a gel in a fragmented state. It can be confirmed that a gel surface area is widened when the gel is not formed through agglomeration, but is formed in a fragmented state. This may be the cause of a high initial release rate of the drug.

On the other hand, since the composition of Example 10 according to the present invention is a form already containing water, it can be confirmed that the composition is rapidly converted into a gel in the tissue and agglomerated so that a gel is not formed. It can be seen that the composition of Example 10 according to the present invention may provide a low initial release rate of the drug.

### [Experimental Example 4] Confirmation of PK of injectable composition in guinea pig

### [Experimental Example 4-1] Confirmation of PK of injectable composition of Example 13, and Comparative Example 3 in guinea pig

A pharmacokinetics of the compositions of the present invention was confirmed in guinea pigs through the following experiment. Using a disposable syringe, the compositions of Comparative Examples 3 and Example 13 were subcutaneously injected into the back of six-week-old male guinea pigs (n=4) which weighed 500 g on average so that an administration weight of leuprolide may reach 3.75 mg/head (equivalent to one month as a human dose). A guinea pig is an animal with developed subcutaneous fat like humans, and an attempt was made to confirm a PK profile (pharmacokinetic profile) in adipose tissues.

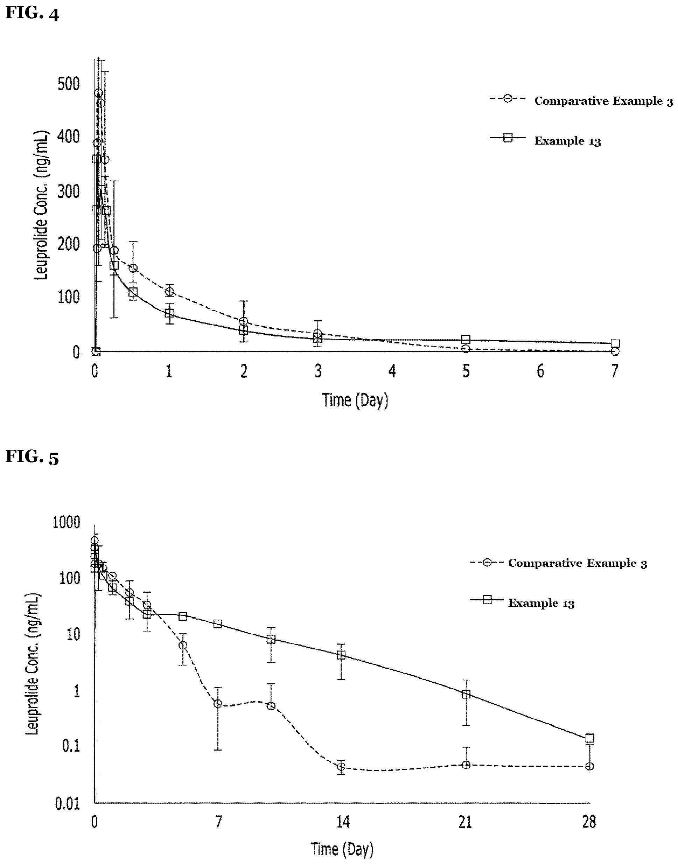

The concentration of leuprolide in a plasma sample of guinea pigs was analyzed with regard to a PK profile for 28 days by using the liquid chromatography/mass spectrometer (LC-MS). The results of [FIG. 4] and [FIG. 5] show an average value for four guinea pigs used in the experiment. [FIG. 4] is to confirm a difference in the initial release rate of the drug, while [FIG. 5] shows a log transformation result in order to confirm a difference in drug blood concentration in guinea pigs in a later half.

Referring to FIG. 4, in the case of the composition of Example 13 containing water, it was confirmed that a liquid formulation is rapidly converted into a gel in vivo, thereby significantly lowering an initial release rate. For the composition of Comparative Example 3, it can be confirmed that an area under the curve (AUC) up to three days is 7864 µg hr/mL. However, for the composition of Example 13, it can be also confirmed that the AUC up to three days is 5528 µg hr/mL, which is about 30% reduced compared to Comparative Example 3.

Referring to FIG. 5, it can be confirmed that the composition of Example 13 shows a decline in an initial drug dumping phenomenon and maintains a drug blood concentration higher than the composition of Comparative Example 3 for one month while the drug remains in the gel formulation.

### [Experimental Example 4-2] Confirmation of PK of injectable composition of Example 19 in guinea pig

Using the liquid injectable formulation prepared in Example 19 according to the present invention, a pharmacokinetics of Example 19 on guinea pigs was confirmed. Using a disposable syringe, the composition of Example 19 was subcutaneously injected into the back of six-week-old male guinea pigs (n=4) which weighed 500 g on average so that an administration weight of leuprolide may reach 3.75 mg/head (equivalent to one month as a human dose). Here, a guinea pig is an animal with developed subcutaneous fat like humans, and an attempt was made to confirm a PK profile (pharmacokinetic profile) in adipose tissues.

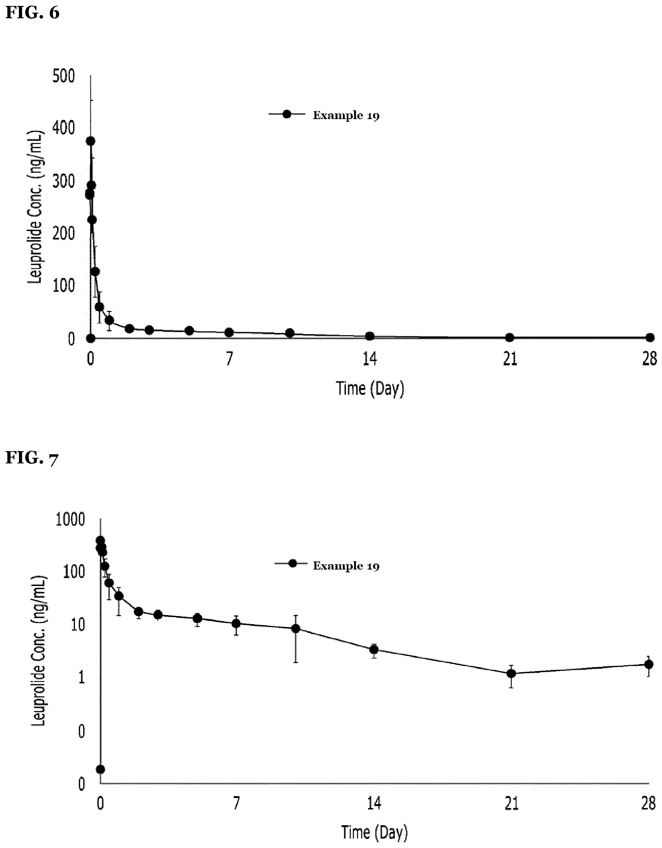

The concentration of leuprolide in a plasma sample of guinea pigs was analyzed with regard to a PK profile for 28 days by using the liquid chromatography/mass spectrometer (LC-MS). The results of [FIG. 6] and [FIG. 7] show an average value for four guinea pigs used in the experiment. [FIG. 6] is to confirm a difference in the initial release rate of the drug, while [FIG. 7] shows a log transformation result in order to confirm a difference in drug blood concentration in guinea pigs in a later half.

The results of [FIG. 6] and [FIG. 7] show the guinea pig PK results of Example 19 a liquid formulation containing 10.5% of water, and it was confirmed that an initial dumping of the drug is low compared with the PK result value of Comparative Example 3 of Experimental Example 4-1, and it was confirmed that a drug concentration is maintained higher than that of Comparative Example 3 even in 28 days later.

Through above Experimental Examples 4-1 and 4-2, in the case of the liquid injectable formulations containing water of Examples 19 and 13, it was considered that an initial concentration of the drug is lower than that of the formulation of Comparative Example 3 which does not contain water, and thus safety is more advantageous. And, it was confirmed that a sustained-release property of the drug is improved because the concentration of the drug is maintained higher than that of the composition of Comparative Example 3 even in 28 days later.

### [Experimental Example 5] Confirmation of PK of injectable composition in humans

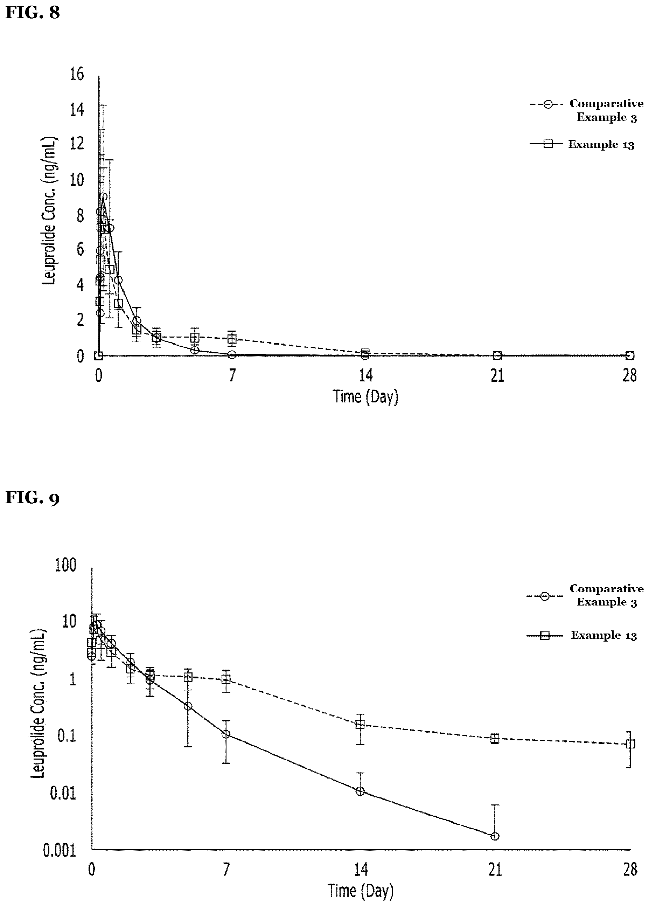

For each of the compositions of Comparative Example 3 and Example 13, a a pharmacokinetics of Example 13 in human was confirmed. For healthy postmenopausal women, 3.75 mg of Comparative Example 3 and Example 13 as leuprolide was subcutaneously administered to the abdomen of six patients per test group. The concentration of leuprolide in a plasma sample of humans was analyzed with regard to a PK profile for 28 days by using the liquid chromatography/mass spectrometer (LC-MS). The PK profiles of Comparative Example 3 and Example 13 in humans were as shown in [FIG. 8] and [FIG. 9]. [FIG. 8] is to confirm a difference in the initial drug release rate, and [FIG. 9] shows a log-transformation to confirm a difference in the drug blood concentration of the leuprolide drug in the second half.

Referring to FIG. 8, in the PK profile in humans similar to that of the guinea pig, it was confirmed that a drug dumping phenomenon immediately after administration is remarkably lowered in the composition of Example 13 compared to the composition of Comparative Example 3.

For the composition of Comparative Example 3, it was found that an area under the curve (AUC) up to three days after administration is 276 µg hr/mL. However, for that of Example 13, it was found that the AUC up to three days is 209 µg hr/mL, which is about 25% lower than the composition of Comparative Example 3.

Referring to FIG. 9, it was found that the composition of Example 13 shows a decline in an initial drug dumping phenomenon and maintains a drug blood concentration higher than the composition of Comparative Example 3 for one month while the drug remains in the gel formulation.

## Claims

1. An injectable composition comprising:
a) a sorbitan unsaturated fatty acid ester comprising two or more -OH (hydroxyl) groups in a polar head group;
b) a phospholipid;
c) a liquid crystal hardener having no ionized group, and a hydrophobic portion thereof having a triacyl group comprising 15 to 40 carbon atoms or a carbon ring structure;
d) water; and
e) a gonadotropin-releasing hormone (GnRH) analogue as pharmaceutically active substance,
and wherein the injectable composition is liquid before injection and forms a liquid crystal after injection.

2. The injectable composition according to claim 1, wherein the sorbitan unsaturated fatty acid ester is selected from the group consisting of sorbitan monooleate, sorbitan monolinoleate, sorbitan monopalmitoleate, sorbitan monomyristoleate, sorbitan sesquioleate, sorbitan sesquilinoleate, sorbitan sesquipalmitoleate, sorbitan sesquimyristoleate, sorbitan dioleate, sorbitan dilinoleate, sorbitan dipalmitoleate, sorbitan dimyristoleate, and mixtures thereof.

3. The injectable composition according to claim 1, wherein the sorbitan unsaturated fatty acid ester is selected from the group consisting of sorbitan monooleate, sorbitan sesquioleate, sorbitan monolinoleate, sorbitan monopalmitoleate, sorbitan monomyristoleate, sorbitan sesquioleate, and mixtures thereof.

4. The injectable composition according to claim 1, wherein the phospholipid is selected from the group consisting of phosphatidylcholine, phosphatidylethanolamine, phosphatidylserine, phosphatidylglycerine, phosphatidylinositol, phosphatidic acid, sphingomyelin, and mixtures thereof, which have 4 to 30 saturated or unsaturated carbon atoms.

5. The injectable composition according to claim 4, wherein the phospholipid is phosphatidylcholine.

6. The injectable composition according to claim 1, wherein the liquid crystal hardener is selected from the group consisting of triglyceride, retinyl palmitate, tocopherol acetate, cholesterol, benzyl benzoate, ubiquinone, and mixtures thereof.

7. The injectable composition according to claim 1, wherein the liquid crystal hardener is selected from the group consisting of tocopherol acetate, cholesterol, and mixtures thereof.

8. The injectable composition according to claim 1, wherein the water is added as at least one selected from the group consisting of water for injection, distilled water, and buffer.

9. The injectable composition according to claim 1, wherein the GnRH analogue is a GnRH agonist or a GnRH antagonist.

10. The injectable composition according to claim 9, wherein the GnRH agonist is selected from the group consisting of leuprolide, goserelin, triptorelin, nafarelin, buserelin, histrelin, deslorelin, meterelin, gonadrelin, pharmaceutical acceptable salts thereof, and mixtures thereof.

11. The injectable composition according to claim 9, wherein the GnRH antagonist is selected from the group consisting of degarelix, abarelix, ganirelix, cetrorelix, pharmaceutical acceptable salts thereof, and mixtures thereof.

12. The injectable composition according to claim 1, wherein the GnRH analogue is selected from the group consisting of leuprolide, goserelin, triptorelin, degarelix, abarelix, pharmaceutical acceptable salts thereof, and mixtures thereof.

13. The injectable composition according to claim 1, wherein the GnRH analogue is leuprolide or pharmaceutical acceptable salts thereof.

14. The injectable composition according to claim 1, wherein the injectable composition is used for preventing or treating sex hormone-dependent diseases, or for contraception.

15. The injectable composition according to claim 14, wherein the sex hormone-dependent disease is prostate cancer, breast cancer, ovarian cancer, endometriosis, uterine fibroid, polycystic ovarian disease, precocious puberty, hypertrichosis, gonadotroph pituitary adenomas, sleep apnea syndrome, irritable bowel syndrome, premenstrual syndrome, benign prostatic hyperplasia, or infertility.

16. The injectable composition according to claim 1, wherein a weight ratio of component a) and component b) is 10:1 to 1:10.

17. The injectable composition according to claim 1, wherein a weight ratio of components a) + b); and component c) is 1000:1 to 1:1.

18. The injectable composition according to claim 1, wherein a weight ratio of components a) + b) + c); and component d) is 99:1 to 1:1.

19. The injectable composition according to claim 1, wherein a weight ratio of components a) + b) + c) + d); and component e) is 10000:1 to 1:1.

20. The injectable composition according to claim 1, comprising:
a) 9 to 90 wt% of the sorbitan unsaturated fatty acid ester having two or more - OH (hydroxyl) groups in a polar head group;
b) 9 to 90 wt% of the phospholipid;
c) 0.1 to 50 wt% of the liquid crystal hardener which does not have an ionized group, and of which a hydrophobic portion has the triacyl group having 15 to 40 carbon atoms or a carbon ring structure;
d) 0.5 to 50 wt% of the water; and
e) 0.01 to 50 wt% of gonadotropin-releasing hormone (GnRH) analogue.

21. The injectable composition according to claim 1, comprising:
a) 9 to 50 wt% of the sorbitan unsaturated fatty acid ester comprising two or more -OH (hydroxyl) groups in a polar head group;
b) 18 to 60 wt% of the phospholipid;
c) 1 to 36 wt% of the liquid crystal hardener which does not have an ionized group, and of which a hydrophobic portion has the triacyl group comprising 15 to 40 carbon atoms or a carbon ring structure;
d) 0.5 to 10.5 wt% or 25 to 37.5 wt% of the water; and
e) 0.1 to 45 wt% of leuprolide or pharmaceutically acceptable salts thereof.

22. The injectable composition according to claim 1, comprising:
a) 9 to 50 wt% of the sorbitan unsaturated fatty acid ester comprising two or more -OH (hydroxyl) groups in a polar head group;
b) 18 to 60 wt% of the phospholipid;
c) 1 to 36 wt% of the liquid crystal hardener which does not have an ionized group, and of which a hydrophobic portion has the triacyl group comprising 15 to 40 carbon atoms or a carbon ring structure;
d) 0.5 to 10.5 wt% or 25 to 37.5 wt% of the water; and
e) 0.1 to 45 wt% of goserelin or pharmaceutically acceptable salts thereof.

23. The injectable composition according to claim 1, comprising:
a) 9 to 50 wt% of the sorbitan unsaturated fatty acid ester comprising two or more -OH (hydroxyl) groups in a polar head group;
b) 18 to 60 wt% of the phospholipid;
c) 1 to 36 wt% of the liquid crystal hardener which does not have an ionized group, and of which a hydrophobic portion has the triacyl group comprising 15 to 40 carbon atoms or a carbon ring structure;
d) 0.5 to 10.5 wt% or 25 to 37.5 wt% of the water; and
e) 0.1 to 45 wt% of degarelix or pharmaceutically acceptable salts thereof.

24. A method for preventing or treating sex hormone-dependent diseases, or for contraception, the method, comprising administering a therapeutically effective amount of the injectable composition according to claim 1 into a subject.

25. Use of the injectable composition according to claim 1 for preventing or treating sex hormone-dependent diseases, or for contraception.

26. Use of the injectable composition according to claim 1, in the manufacture of a medication for preventing or treating sex hormone-dependent diseases, or for contraception.
